# EUROPEAN PATENT APPLICATION

(11) **EP 1 211 323 A1**
(43) Date of publication of application: **05.06.2002**
(21) Application number: 00204322.2
(22) Date of filing: 04.12.2000
(51) Int. Cl.: C12Q 1/68

(54) **Tests based on nucleic acids of endosymbiont cellular organelles**

(71) Applicant: Amsterdam Support Diagnostics B.V., 1105 BA Amsterdam (NL)
(72) Inventor: Van Gemen, Bob, 1326 HV Almere (NL); Timmermans, Eveline Catherina Anna Clasina, 5231 HS's-Hertogenbosch (NL); de Ronde, Anthonij, 1107 DE Amsterdam (NL); Dobbelaer, Irene Johanna Monica, 5242 Rosmalen (NL)
(74) Representative: Prins, Adrianus Willem

(57) **Abstract**

The invention relates to the diagnosis of disease or the determination of functioning of cellular organisms, being of multi-cellular or unicellular nature, being visible by the naked eye or being a micro-organism. The invention provides a method for determining functioning of a cellular organism comprising determining the relative ratio of a first endosymbiont cellular organelle nucleic acid and/or gene product thereof in a sample obtained from said organism.

### Annex to the application documents - subsequently filed sequences listing

SEQUENCE LISTING

<110> Amsterdam Support Diagnostics B.V.

<120> Testing endosymbiont cellular organelles and compounds
      identifiable therewith

<130> P53526EP00

<140> 00204322.2
<141> 2000-12-04

<160> 31

<170> PatentIn Ver. 2.1

<210> 1
<211> 48
<212> DNA
<213> Artificial Sequence

<220>
<223> Description of Artificial Sequence: primer MtD p1

<220>
<221> misc_feature
<222> (1)..(48)

<400> 1
aattctaata cgactcacta tagggagaag agccgttgag ttgtggta          48


<210> 2
<211> 25
<212> DNA
<213> Artificial Sequence

<220>
<223> Description of Artificial Sequence: primer MtD p2

<220>
<221> misc_feature
<222> (1)..(25)

<400> 2
tctccatcta ttgatgaggg tctta          25


<210> 3
<211> 34
<212> DNA
<213> Artificial Sequence

<220>
<223> Description of Artificial Sequence: probe MtD mb

<220>
<221> misc_feature
<222> (1)..(34)

<400> 3
gcatgcccct cctagcctta ctactaatgc atgc          34


<210> 4
<211> 49
<212> DNA
<213> Artificial Sequence

<220>
<223> Description of Artificial Sequence: primer SnrpD
      p1

<220>
<221> misc_feature
<222> (1)..(49)

<400> 4
aattctaata cgactcacta tagggagagg cccggcatgt ggtgcataa                49


<210> 5
<211> 23
<212> DNA
<213> Artificial Sequence

<220>
<223> Description of Artificial Sequence: primer SnrpD
      p2

<220>
<221> misc_feature
<222> (1)..(23)

<400> 5
ttccttacat ctctcacccg cta                                            23


<210> 6
<211> 32
<212> DNA
<213> Artificial Sequence

<220>
<223> Description of Artificial Sequence: probe SnrpD mb

<220>
<221> misc_feature
<222> (1)..(32)

<400> 6
gcatgctgta accacgcact ctcctcgcat gc                                  32


<210> 7
<211> 20
<212> DNA
<213> Artificial Sequence

<220>
<223> Description of Artificial Sequence: primer SnrpD2
      p2

<220>
<221> misc_feature
<222> (1)..(20)

<400> 7
tgcgcctctt tctgggtgtt                                                20


<210> 8
<211> 47
<212> DNA
<213> Artificial Sequence

<220>
<223> Description of Artificial Sequence: primer MtR p1

<220>
<221> misc_feature
<222> (1)..(47)

<400> 8
aattctaata cgactcacta tagggaggag aagatggtta ggtctac                47


<210> 9
<211> 24
<212> DNA
<213> Artificial Sequence

<220>
<223> Description of Artificial Sequence: primer MtR p2

<220>
<221> misc_feature
<222> (1)..(24)

<400> 9
cgatatggcg ttcccccgca taaa                                         24


<210> 10
<211> 34
<212> DNA
<213> Artificial Sequence

<220>
<223> Description of Artificial Sequence: probe MtR mb

<220>
<221> misc_feature
<222> (1)..(34)

<400> 10
gctccgaagc ttctgactct tacctccccg gagc                              34


<210> 11
<211> 49
<212> DNA
<213> Artificial Sequence

<220>
<223> Description of Artificial Sequence: primer SnrpR
      p1

<220>
<221> misc_feature
<222> (1)..(49)

<400> 11
aattctaata cgactcacta tagggagagg cccggcatgt ggtgcataa              49


<210> 12
<211> 23
<212> DNA
<213> Artificial Sequence

<220>
<223> Description of Artificial Sequence: primer SnrpR
      p2

<220>
<221> misc_feature
<222> (1)..(23)

<400> 12
cagtatgcca agaccgactc aga                                              23


<210> 13
<211> 34
<212> DNA
<213> Artificial Sequence

<220>
<223> Description of Artificial Sequence: probe SnrpR mb

<220>
<221> misc_feature
<222> (1)..(34)

<400> 13
cgtacgagaa gaggaagccc aagagccacg tacg                                  34


<210> 14
<211> 49
<212> DNA
<213> Artificial Sequence

<220>
<223> Description of Artificial Sequence: primer
      Rubisco-DNA p1

<220>
<221> misc_feature
<222> (1)..(49)

<400> 14
aattctaata cgactcacta tagggggata atttcattac cttcacgag                  49


<210> 15
<211> 21
<212> DNA
<213> Artificial Sequence

<220>
<223> Description of Artificial Sequence: primer
      Rubisco-DNA p2

<220>
<221> misc_feature
<222> (1)..(21)

<400> 15
ggagtcctga actagccgca g                                                21


<210> 16
<211> 34
<212> DNA
<213> Artificial Sequence

<220>
<223> Description of Artificial Sequence: probe
      Rubisco-DNA MB

<220>
<221> misc_feature
<222> (1)..(34)

<400> 16
gcatgcggta gataaactag atagctaggc atgc                                34


<210> 17
<211> 46
<212> DNA
<213> Artificial Sequence

<220>
<223> Description of Artificial Sequence: primer
      Rubisco-RNA p1

<220>
<221> misc_feature
<222> (1)..(46)

<400> 17
aattctaata cgactcacta taggggagtt gttgttattg taagtc                   46


<210> 18
<211> 22
<212> DNA
<213> Artificial Sequence

<220>
<223> Description of Artificial Sequence: primer
      Rubisco-RNA p2

<220>
<221> misc_feature
<222> (1)..(22)

<400> 18
caagtcttat gaattcctat ag                                             22


<210> 19
<211> 32
<212> DNA
<213> Artificial Sequence

<220>
<223> Description of Artificial Sequence: probe
      Rubisco-RNA-MB

<220>
<221> misc_feature
<222> (1)..(32)

<400> 19
gctagcacac agggtgtacc cattatgcta gc                                  32


<210> 20
<211> 48
<212> DNA
<213> Artificial Sequence

<220>
<223> Description of Artificial Sequence: primer
      OryzaDNA p1

<220>
<221> misc_feature
<222> (1)..(48)

<400> 20
aattctaata cgactcacta tagggggatc ttaattacat gccgttca                48


<210> 21
<211> 20
<212> DNA
<213> Artificial Sequence

<220>
<223> Description of Artificial Sequence: primer
      OryzaDNA p2

<220>
<221> misc_feature
<222> (1)..(20)

<400> 21
aaaggtgccg gttctcacta                                               20


<210> 22
<211> 36
<212> DNA
<213> Artificial Sequence

<220>
<223> Description of Artificial Sequence: probe OryzaDNA
      mb

<220>
<221> misc_feature
<222> (1)..(36)

<400> 22
gctagcctct gcaagcttca tcagtaatag gctagc                             36


<210> 23
<211> 51
<212> DNA
<213> Artificial Sequence

<220>
<223> Description of Artificial Sequence: primer
      OryzaRNA p1

<220>
<221> misc_feature
<222> (1)..(51)

<400> 23
aattctaata cgactcacta taggggctaa tgcccttttc ttttcttcct c             51


<210> 24
<211> 20
<212> DNA
<213> Artificial Sequence

<220>
<223> Description of Artificial Sequence: primer
      OryzaRNA p2

<220>
<221> misc_feature
<222> (1)..(20)

<400> 24
catattggct ttcgaagatt                                                    20


<210> 25
<211> 36
<212> DNA
<213> Artificial Sequence

<220>
<223> Description of Artificial Sequence: probe OryzaRNA
      mb

<220>
<221> misc_feature
<222> (1)..(36)

<400> 25
gctagccttc agccattatt caagatggtg gctagc                                  36


<210> 26
<211> 49
<212> DNA
<213> Artificial Sequence

<220>
<223> Description of Artificial Sequence: primer
      tRNA-L-D p1

<220>
<221> misc_feature
<222> (1)..(49)

<400> 26
aattctaata cgactcacta taggggggtt ctagttcgag aaccgcttg                     49


<210> 27
<211> 21
<212> DNA
<213> Artificial Sequence

<220>
<223> Description of Artificial Sequence: primer
      tRNA-L-D p2

<220>
<221> misc_feature
<222> (1)..(21)

<400> 27
gcgaaatcgg tagacgctac g                                                  21


<210> 28
<211> 32
<212> DNA
<213> Artificial Sequence

<220>
<223> Description of Artificial Sequence: probe tRNA-L-D
      MB

<220>
<221> misc_feature
<222> (1)..(32)

<400> 28
gctagccaac ttccaaattc agagaagcta gc                                32


<210> 29
<211> 48
<212> DNA
<213> Artificial Sequence

<220>
<223> Description of Artificial Sequence: primer petB
      RNA p1

<220>
<221> misc_feature
<222> (1)..(48)

<400> 29
aattctaata cgactcacta tagggaaacc ggtagcaact tgtactag               48


<210> 30
<211> 25
<212> DNA
<213> Artificial Sequence

<220>
<223> Description of Artificial Sequence: primer petB
      RNA p2

<220>
<221> misc_feature
<222> (1)..(25)

<400> 30
ggtttcggta tctctggaat atgag                                        25


<210> 31
<211> 32
<212> DNA
<213> Artificial Sequence

<220>
<223> Description of Artificial Sequence: probe petB RNA
      MB

<220>
<221> misc_feature
<222> (1)..(32)

<400> 31
gctagcgagg aacgtcttga gattcagcta gc                                32

## Description

The invention relates to the diagnosis of disease or the determination of functioning of cellular organisms, being of multi-cellular or unicellular nature, being visible by the naked eye or being a micro-organism.

A diagnostician of disease is a student of (mal)functioning of cellular organisms. Said student classically can employ a broad range of inroads into said organism to obtain relevant information as to the various aspects of said malfunctioning. These inroads vary widely, examples range from detecting relative ratios of kidney stones by studying urinary samples obtained from various patients, from probing for the presence or absence of intestinal ulcers via endoscopy, from scanning for detectable tumors by nuclear magnetic resonance (NMR), from detecting diabetes by testing for insulin levels and/or glucose concentration in blood plasma, from determining cancer proneness by determining transcriptional levels of oncogenes, and so on.

Currently, detection of disease or malfunctioning (or vice versa, of health and proper functioning) of higher organisms, such as animals and plants relies very much on testing samples obtained from these organisms and studying these samples in a laboratory Often when a fruitful method capable of determining, identifying or detecting (aspects of) a disease of malfunctioning of an organism has been found it is in general also useful in testing or screening for compounds or methods useful for treatment of (aspects of) said disease or malfunctioning or useful in testing or screening for compounds or methods involved in causing (aspects of) said disease or malfunctioning; by using the same or a similar method as used in diagnosis it is generally possible to generate an assessment of the usefulness of such candidate compounds or methods in treating and/or causing the disease or malfunctioning in question. Clearly, life science laboratories are always in the need of yet other inroads into organisms to obtain yet more information relating to disease or malfunctioning and to compounds and methods related to cause and/or treatment of disease or malfunctioning.

The invention provides a method for determining (mal)functioning of an cellular organism comprising determining the relative ratio of an endosymbiont cellular organelle nucleic acid and/or or gene product thereof in relation to another nucleic acid or gene product present in a sample obtained from said organism. Endosymbiont cellular organelles are those organelles of an eukaryotic cell that are thought to have been derived of prokaryotic bacteria very early on in the evolution of eukaryotic cells; these bacteria (is thought) have engaged in an symbiosis with early eukaryotic cells, and at present, eukaryotic cells comprising these endosymbiont organelles in general cannot live without them; none of the present eukaryotic cells would function properly without mitochondria, and most plant cells would at least considered to be malfunctioning when no proplastids, or organelles derived thereof, such as chloroplasts, etioplasts, amyloplasts, elaioplasts or chromoplasts were present. These organelles in general appear to be at least partially self-replicating bodies which, although under some nuclear controls, still possess considerable autonomy.

In particular, the invention provides a method whereby said relative ratio of an endosymbiont cellular organelle nucleic acid and/or or gene product thereof is determined in relation to the amount of essentially nuclear nucleic acid detectable in said sample (be it DNA or RNA), or in relation to gene products (derivable by transcription and/or translation, such as mRNA or (poly)peptides) of said nuclear nucleic acid, (nuclear nucleic acid herein comprises chromosomal DNA and the RNA transcribed therefrom) for example present in nuclear or cytoplasmatic fractions or parts of said sample. DNA or corresponding mRNA encoding components of small nuclear ribonucleoprotein (SNRNP), or other essentially common nucleic acid derived from chromosomal DNA, is particularly useful to test, because of its ubiquitous presence. In this way, the invention provides a method for studying for example mitochondrial and/or proplastid related disease. Endosymbiont cellular organelle related disease encoded by defects in said organelle's DNA manifests in many different syndromes and is often variable in its expression (and thus in general hard to detect by testing for clinical parameters alone) due to heteroplasmy, whereby mutant and wild type nucleic acid can be found in one cell, whereby its distribution can vary. Endosymbiont cellular organelle related disease is often aggravated with increasing age of the affected individual. Endosymbiont cellular organelle related disease can also often be observed after treatment against other disease with various drugs, and than contributes to various side-effects of those drugs that basically one would like to avoid during treatment. Those side effects can now be better studied by using a method as provided herein.

Furthermore, the invention provides a method whereby said relative ratio of an endosymbiont cellular organelle nucleic acid and/or or gene product thereof is determined in relation to the amount of a distinct endosymbiont cellular organelle acid detectable in said sample (be it DNA or RNA), or in relation to gene products (derivable by transcription and/or translation, such as mRNA or (poly)peptides) of said endosymbiont cellular organelle nucleic acid. In one aspect of the invention the method involves determining a ratio between organelle DNA, such as mtDNA, and the corresponding transcriptionally derivable organelle RNA, in the example the related mtRNA, or translated gene product. In yet another aspect of the invention, the ratio between two distinct organelle DNA's or related gene products is determined.

Furthermore, the invention provides a diagnostic kit comprising at least one means for performing a method according to the invention, said kit comprising at least one primer or probe set selective for the amplification and detection of a nucleic acid related to or derived from endosymbiont cellular organelles and, when so desired, necessary amplification reagents, such as can be found examplified in the detailed description herein or otherwise known in the art. In particular, the invention provides a diagnostic kit wherein said kit comprises more than one primer or probe set for the amplification of nucleic acid sequences related to cellular organelles, preferably supplemented with a primer or probe set for the amplification of nucleic acid related to the chromosomes, such as a SNRNP specific primer or probe. In particular the invention provides a kit comprising at least one primer or probe from table 1 for the amplification of nucleic acid sequences related to cellular organelles. It is of course preferred that said amplification reagents, when provided with the kit, comprise an enzyme with reverse transcriptase activity, such as required for PCR or NASBA amplification. Of course, a kit comprising a means for the detection of a geneproduct other than nucleic acid, for use in a method according to the invention is herewith also provided.

Other possible uses of the invention lay in candidate drug testing, for beneficial activity and/or side effects of possible medicaments or pharmaceutical compositions such as candidate anti-parasitic compounds, antibiotic compounds, cytostatic compounds, and so on. For example, the invention provides a method for determining therapeutic activity and/or possible side-effects of a candidate compound, for example in determining its usefulness for treatment of malfunctioning of a cellular organism, comprising determining the relative ratio of an endosymbiont cellular organelle nucleic acid and/or or gene product thereof in a sample obtained from an organism, preferably said organism or an essentially related organism, such as belonging to the same species or genus, having been provided with said compound. Also use for (selective) toxin testing, of e.g. herbicides, insecticides, anti-parasitic compounds, antibiotic compounds is here provided, the invention provides a method for determining toxic activity of a candidate compound, for example in determining its usefulness for causing malfunctioning of a cellular organism, e.g. by having a cytostatic or even cytotoxic effect, comprising determining the relative ratio of an endosymbiont cellular organelle nucleic acid and/or or gene product thereof in a sample obtained from an organism, preferably said organism or related organism, having been provided with said compound.

In a preferred embodiment, selectivity is also tested, using or applying the method as provided herein (preferably in parallel experiments) on or to a first organism and on or to an essentially unrelated second organism, if desired belonging to a different family or order, but preferably belonging to at least a different class or phylum, most preferably belonging to a different kingdom of organisms. Selectivity aspects are for example tested by testing the compounds in (if desired only in cells of) a first target organism (such as a bacterium or parasite) as well as of testing the host or cells thereof, being an essentially unrelated second organism, for example a mammal or plant, or by testing of a crop plant or cells thereof as well as testing an essentially unrelated weed plant or cells thereof with said compound, to determine for example selective toxic or selective therapeutic effects. It is also provided to test normal cells derived from an individual in parallel or comparison with aberrant cells, such as tumour cells derived from the same individual, to detect or screen for a tumour-specific or at least selective cytostatic or cytotoxic compound for use in therapy of said individual or others with similar or related disease.

The invention furthermore provides the use of a compound obtainable or detectable by a method according to the invention in the preparation of a medicament, a herbicide, insecticide, anti-parasiticum, cytostatic, etc, and a medicament, herbicide, insecticide, anti-parasiticum etc. obtainable or derivable or identifiable by a method according to the invention.

The invention is further explained in the detailed description herein, wherein most examples are directed by way of example at testing of mitochondriae, being central to the provision and use of energy in a cell, however, it will easily be understood that the same principles apply to tests using other endosymbiont organelles, such as chloroplasts, being central to the provision of carbohydrates to a plant cell.

### Figure legends

Figure 1. Examples of standard curves for DNA and RNA target sequences.

Figure 2. Ratio of mitochondrial DNA and chromosomal DNA in fibroblast cells cultured in the presence of DDC.

Figure 3. Ratio of mitochondrial RNA and chromosome encode RNA in fibroblast cells cultured in the presence of DDC.

Figure 4. Ratio of mitochondrial RNA and chromosomal DNA in fibroblast cells cultured in the presence of DDC.

Figure 5. Ratio of mitochondrial RNA and mitochondrial DNA in fibroblast cells cultured in the presence of DDC.

Figure 6. Ratio of chromosome encoded RNA and chromosomal DNA in fibroblast cells cultured in the presence of DDC.

Figure 7. Ratio of mitochondrial DNA and chromosomal DNA in fibroblast cells cultured in the absence of DDC after being cultured with DDC for 4 weeks.

Figure 8. Ratio of mitochondrial RNA and chromosome encoded RNA in fibroblast cells cultured in the absence of DDC after being cultured with DDC for 4 weeks.

Figure 9. Ratio of mitochondrial DNA and chromosomal DNA in PBMC's cultured in the presence of DDC for 5 days.

Figure 10. Ratio of mitochondrial RNA and chromosome encoded RNA in PBMC's cultured in the presence of DDC for 5 days.

Figure 11. Comparison of SNRNP DNA NASBA reactions with and without pre-treatment with restriction enzyme Msp I.

### Examples Endosymbiont Cellular Organelle Diagnostics

### Used ingredients and general methodology

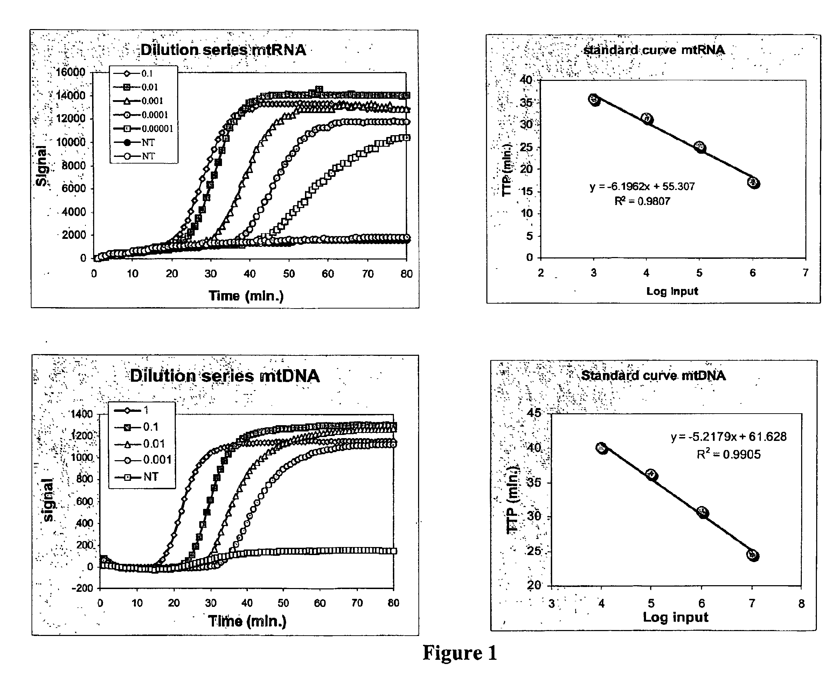

In table 1 the primers and probes used in the examples are summarised.
Standard NASBA nucleic acid amplification reactions were performed in a 20µl reaction volume and contained: 40mM Tris-pH 8.5, 70mM KCl, 12mM MgCl2, 5mM dithiotreitol, 1mM dNTP's (each), 2mM rNTP's (each), 0.2µM primer (each), 0.05µM molecular beacon, 375mM sorbitol, 0.105 µg/µl bovine serum albumin, 6.4 units AMV RT, 32 units T7 RNA polymerase, 0.08 units RNAse H and input nucleic acid. The complete mixture, except the enzymes, sorbitol and/or bovine serum albumin was, prior to adding the enzyme mixture, heated to 65°C for 2 minutes in order to denature any secondary structure in the RNA and to allow the primers to anneal. After cooling the mixture to 41°C the enzymes were added. The amplification took place at 41°C for 90 min in a fluorimeter (CytoFluor 2000) and the fluorescent signal was measured every minute (using the filter set 530/25 nm and 485/30 nm). For amplification of DNA target sequences the 65°C denaturation step was replaced with a 95°C denaturation step for the same period of time.
To achieve quantification, a dilution series of target sequence for a particular primer set was amplified and the time points at which the reactions became positive (the time to positivity, TTP) were plotted against the input amounts of nucleic acid. This way a calibration curve was created that could be used to read TTP values of reactions with unknown amounts of input and deduce the input amount. Examples of typical standard curves for quantification of RNA and DNA are shown in figure 1. For some of the target sequences no dilution series were available with reliable absolute amount of copies determined. Those series were given an arbitrary unit as measurement instead of DNA or RNA copies, e.g. cell-equivalent or ET-unit. As a result it sometimes seems that there is less RNA than DNA, which is quite the opposite of what is expected.
Cells (fibroblasts and PBMC's) were cultured under standard conditions in standard media known to persons skilled in the art with addition of drugs or putative toxic or stimulating compounds as defined in the examples. Nucleic acids were isolated from the cells with the method described by Boom et al (Boom R, Sol CJ, Salimans MM, Jansen CL, Wertheim-van Dillen PM, van der Noordaa J, 1990. Rapid and simple method for purification of nucleic acids. *J Clin Microbiol*; 28(3):495-503) or with dedicated isolation kits purchased from Qiagen (Qiagen GmbH, Max Volmer Strasse 4, 40724 Hilden, Germany) and used according to the manufacturer's protocols. A small aliquot of the isolated nucleic acid was analysed on an agarose gel and the remainder stored at -80°C until further analysis. Usually the nucleic acid was diluted 10 times with water and of the diluted nucleic acid usually 5 µl was used as input in the NASBA amplification reactions.

### Example 1

In this example it is explained which ratio's can be measured with a method according to the invention and the meaning they can have in diagnostic sense:
The invention for example provides determining the relative ratio of organelle DNA to chromosomal DNA. This ratio, when compared with normal values or determined at at least two points in time, shows the decline or increase of organelles per cell. Also is provided determining the ratio of organelle RNA to chromosome encoded RNA. This ratio when compared with normal values or determined at at least two points in time, shows the organelle transcription activity decline or increase per cell, normalised for the active state (i.e. transcription state) of the cell.
Also is provided determining the ratio of organelle RNA to chromosomal DNA. This ratio when compared with normal values or determined at at least two points in time, shows the organelle transcription activity decline or increase per cell
Also is provided determining the ratio of organelle DNA to organelle RNA. This ratio, when compared with normal values or determined at at least two points in time, shows the decline or increase of transcription in the organelle, indicating regulation at the transcriptional level to achieve a certain mRNA (and therefore protein) level.
Also is provided determining the ratio of organelle DNA to chromosome encoded RNA. This ratio, when compared with normal values or determined at at least two points in time, shows the decline or increase of transcription in the cell, in relation to chromosomal RNA transcription levels, indicating the activity state of the organelle, which is especially useful when chromosomal RNA is determined that encodes an organelle protein or other component thereof.

### Example 2

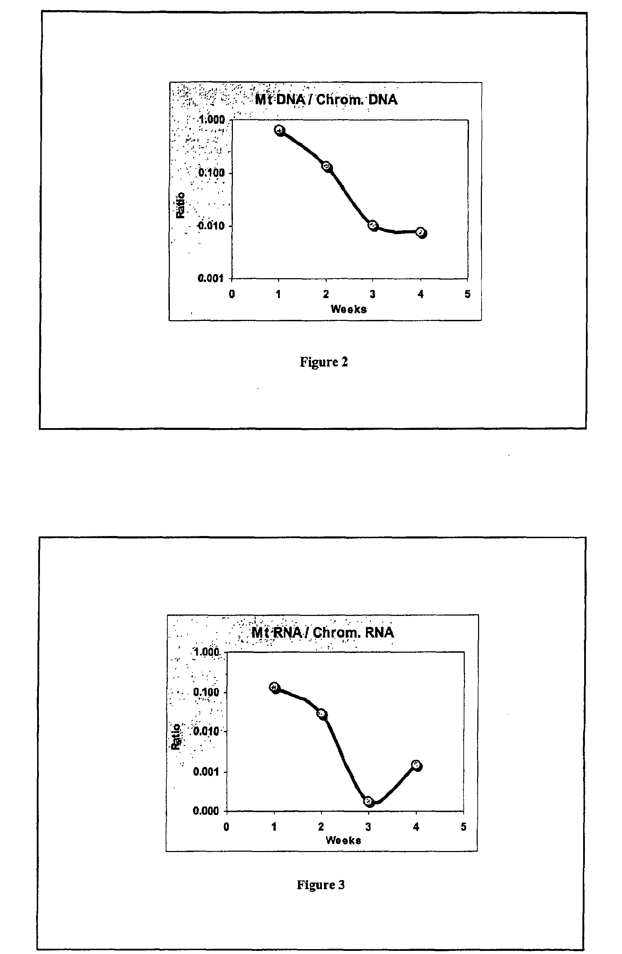

Fibroblast cells were cultured *in vitro* in the presence of the anti viral drugs DDC, AZT and D4T at two concentrations each, 3 µM and 30 µM, respectively, for 4 weeks. As controls cell cultures with ethidium bromide and without drugs were also performed. Ethidium bromide is known to deplete mitochondrial DNA completely from cells and is a positive control in terms of achieving an effect on the mitochondria content of cells. At one week intervals part of the cells was harvested and analyzed for amount of mitochondrial DNA (primers MtD p1 and MtD p2 and probe MtD mb) and chromosomal DNA (primers SnrpD p1 and SnrpD p2 and probe SnrpD mb) in the described NASBA protocol. The cultures with AZT, D4T and without additive showed no measurable change in mitochondrial DNA to chromosomal DNA ratio in the culture period of 4 weeks. The culture with Ethidium bromide showed a decline in mitochondrial DNA content as expected. The results for DDC are shown in figure 2. The data in figure 2 clearly show a decline in the amount of mitochondrial DNA per cell with more than 2 logs and therewith the mitochondrial toxicity of the antiviral drug DDC.

### Example 3

Fibroblast cells were cultured *in vitro* in the presence of the anti viral drugs DDC, AZT and D4T at two concentrations each, 3 µM and 30 µM, respectively, for 4 weeks. As controls cell cultures with ethidium bromide and without drugs were also performed. Ethidium bromide is known to deplete mitochondrial DNA completely from cells and is a positive control in terms of achieving an effect on the mitochondria content of cells. At one week intervals part of the cells was harvested and analyzed for amount of mitochondrial RNA (primers MtR p1 and MtR p2 and probe MtR mb) and chromosome encoded RNA (primers SnrpR p1 and SnrpR p2 and probe SnrpR mb) in the described NASBA protocol. The cultures with AZT, D4T and without additive showed no measurable change in mitochondrial RNA to chromosome encoded RNA ratio in the culture period of 4 weeks. The culture with Ethidium bromide showed a decline in mitochondrial RNA content as expected. The results for DDC are shown in figure 3. The data in figure 3 clearly show a decline in the amount of mitochondrial RNA per cell with at least 2 logs and therewith the mitochondrial toxicity of the antiviral drug DDC. The time point at 3 weeks has a very low value and it presumably this is somewhat of a outlier measurement.

### Example 4

Fibroblast cells were cultured *in vitro* in the presence of the anti viral drugs DDC, AZT and D4T at two concentrations each, 3 µM and 30 µM, respectively, for 4 weeks. As controls cell cultures with ethidium bromide and without drugs were also performed. Ethidium bromide is known to deplete mitochondrial DNA completely from cells and is a positive control in terms of achieving an effect on the mitochondria content of cells. At one week intervals part of the cells was harvested and analyzed for amount of mitochondrial RNA (primers MtR p1 and MtR p2 and probe MtR mb) and chromosomal DNA (primers SnrpD p1 and SnrpD p2 and probe SnrpD mb) in the described NASBA protocol.

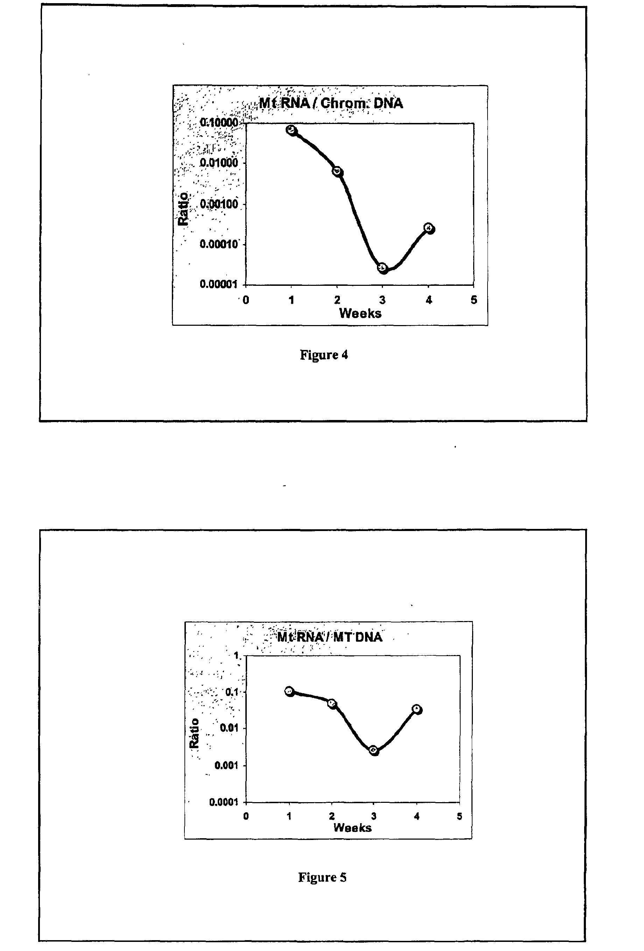

The cultures with AZT, D4T and without additive showed no measurable change in mitochondrial RNA to chromosomal DNA ratio in the culture period of 4 weeks. The culture with Ethidium bromide showed a decline in mitochondrial RNA content as expected. The results for DDC are shown in figure 4.

The data in figure 4 clearly show a decline in the amount of mitochondrial RNA per cell with almost 3 logs and therewith the mitochondrial toxicity of the antiviral drug DDC. The time point at 3 weeks has a very low value and it presumably this is somewhat of a outlier measurement.

### Example 5

Fibroblast cells were cultured *in vitro* in the presence of the anti viral drugs DDC, AZT and D4T at two concentrations each, 3 µM and 30 µM, respectively, for 4 weeks. As controls cell cultures with ethidium bromide and without drugs were also performed. Ethidium bromide is known to deplete mitochondrial DNA completely from cells and is a positive control in terms of achieving an effect on the mitochondria content of cells. At one week intervals part of the cells was harvested and analyzed for amount of mitochondrial RNA (primers MtR p1 and MtR p2 and probe MtR mb) and mitochondrial DNA (primers MtD p1 and MtD p2 and probe MtD mb) in the described NASBA protocol.

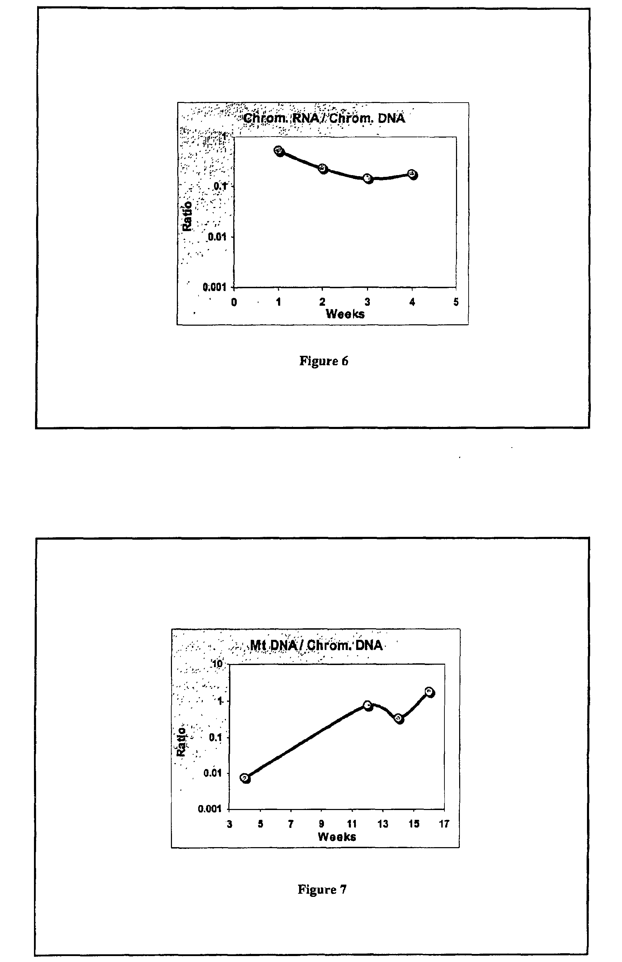

The cultures with AZT, D4T and without additive showed no measurable change in mitochondrial RNA to mitochondrial DNA ratio in the culture period of 4 weeks. The culture with Ethidium bromide showed a decline in mitochondrial RNA and DNA content as expected. The results for DDC are shown in figure 5.

The data in figure 5 clearly show that the ratio of mitochondrial DNA to RNA in not significantly changing over the period of 4 weeks. The time point at 3 weeks in figure 5 has a low value for mitochondrial RNA that shows up, this measurement is presumably somewhat of an outlier measurement.

### Example 6

Fibroblast cells were cultured *in vitro* in the presence of the anti viral drugs DDC, AZT and D4T at two concentrations each, 3 µM and 30 µM, respectively, for 4 weeks. As controls cell cultures with ethidium bromide and without drugs were also performed. Ethidium bromide is known to deplete mitochondrial DNA completely from cells and is a positive control in terms of achieving an effect on the mitochondria content of cells. At one-week intervals part of the cells was harvested and analyzed for amount of chromosome encoded RNA (primers SnrpR p1 and SnrpR p2 and probe SnrpR mb) and chromosomal DNA (primers SnrpD p1 and SnrpD p2 and probe SnrpD mb) in the described NASBA protocol.
The cultures with AZT, D4T, ethidium bromide and without additive showed no measurable change in ratio in the culture period of 4 weeks. The results for DDC are shown in figure 6.
The data in figure 6 clearly show that the ratio of chromosomal DNA to RNA in not significantly changing over the period of 4 weeks.

### Example 7

Fibroblast cells were cultured *in vitro* in the presence of the anti viral drug DDC at a concentration of 30 µM for 4 weeks. After that period the cell culture continued but now in the absence of DDC. During this period of culture without DDC part of the cells was harvested and analyzed for amount of mitochondrial DNA (primers MtD p1 and MtD p2 and probe MtD mb) and chromosomal DNA (primers SnrpD p1 and SnrpD p2 and probe SnrpD mb) in the described NASBA protocol at two-week intervals for a period of 12 weeks. The results of the analysis are shown in figure 7. The results in figure 7 clearly show that the amount of mitochondria per cell increases with more than 2 logs after DDC is removed from the culture. This results shows that the toxic effect of DDC can be reversed if there are still some mitochondria left in the cells to repopulate the new growing cells.

### Example 8

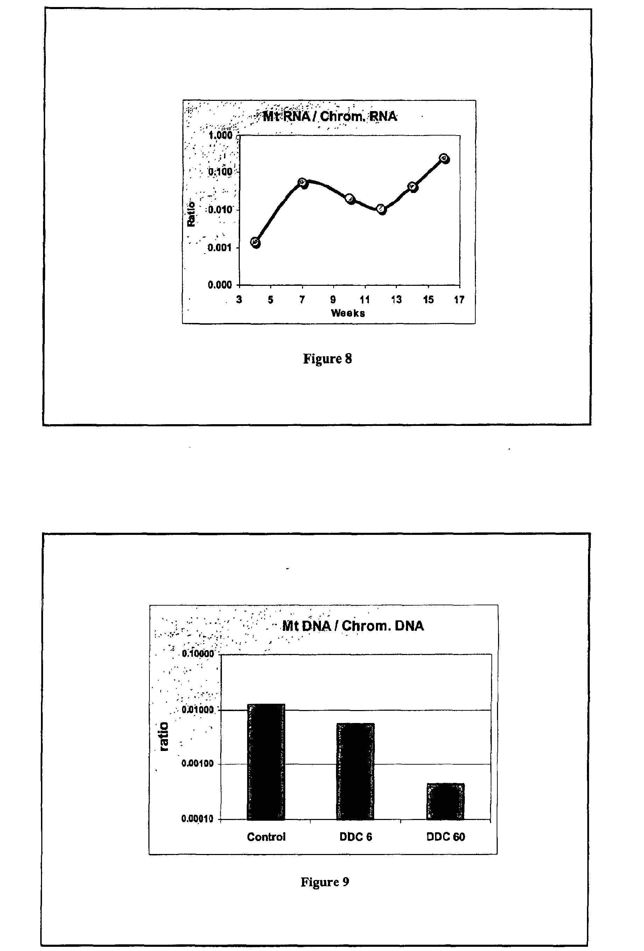

Fibroblast cells were cultured *in vitro* in the presence of the anti viral drug DDC at a concentration of 30 µM for 4 weeks. After that period the cell culture continued but now in the absence of DDC. During this period of culture without DDC part of the cells was harvested and analyzed for amount of mitochondrial RNA (primers MtR p1 and MtR p2 and probe MtR mb) and chromosome encoded RNA (primers SnrpR p1 and SnrpR p2 and probe SnrpR mb) in the described NASBA protocol at two-week intervals for a period of 12 weeks. The results of the analysis are shown in figure 8.
The results in figure 8 clearly show that the amount of mitochondrial RNA per cell increases with more than 2 logs after DDC is removed from the culture. This results shows that the toxic effect of DDC can be reversed and that the function of the mitochondria comes back as shown by synthesis of RNA and subsequently proteins.

### Example 9

Fresh peripheral blood mononuclear cells (PBMC's) from a healthy blood donor were cultured *in vitro* in the presence of the anti viral drugs DDC, AZT and D4T at two concentrations each, 6 µM and 60 µM, respectively, for 5 days. As controls cell cultures with DMSO and without drugs were also performed. DMSO is part of the solvent in which the drugs are solublelized. After 5 days the cells were harvested and analyzed for amount of mitochondrial DNA (primers MtD p1 and MtD p2 and probe MtD mb) and chromosomal DNA (primers SnrpD p1 and SnrpD p2 and probe SnrpD mb) in the described NASBA protocol.

The cultures with AZT, D4T, DMSO and without additive showed no measurable change in ratio in the culture period 5 days. The results for DDC are shown in figure 9.
The results in figure 9 clearly show the decline in PBMC's of mitochondrial DNA per cell of more than 1 log during the 5 day culture period.

### Example 10

Fresh peripheral blood mononuclear cells (PBMC's) from a healthy blood donor were cultured *in vitro* in the presence of the anti viral drugs DDC, AZT and D4T at two concentrations each, 6 µM and 60 µM, respectively, for 5 days. As controls cell cultures with DMSO and without drugs were also performed. DMSO is part of the solvent in which the drugs are solublelized. After 5 days the cells were harvested and analyzed for amount of mitochondrial RNA (primers MtR p1 and MtR p2 and probe MtR mb) and chromosome encoded RNA (primers SnrpR p1 and SnrpR p2 and probe SnrpR mb) in the described NASBA protocol.

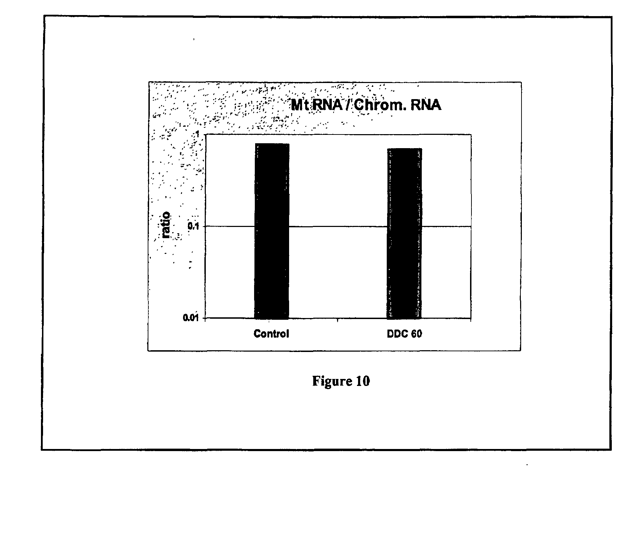

The cultures with AZT, D4T, DMSO and without additive showed no measurable change in ratio in the culture period 5 days. The results for DDC are shown in figure 10. Interestingly, the results in figure 10 do not clearly show a decline in PBMC's of mitochondrial RNA per cell during the 5-day culture period at the highest concentration of DDC used. This is in contrast to the mitochondrial DNA as shown in example 9. It is hypothesized that the decline in mitochondrial DNA is compensated by an increase in transcription, maintaining the level of mitochondrial RNA. This mechanism delays the decline of mitochondrial RNA.
Consequently, one can say that the mitochondrial RNA is a reflection of the current status of the functionality of the mitochondria and that mitochondrial DNA is predictive of what will happen in the (near) future with the mitochondrial function and therefore has a more prognostic character.

### Example 11

Using the primers and probes Rubisco-DNA p1, Rubisco-DNA p2, Rubisco-DNA MB, Rubisco-RNA p1, Rubisco-RNA p2 and Rubisco-RNA-MB (table 1) the chloroplast DNA and RNA of *Oryza sativum* (rice) can be quantified and the ratio to the chromosomal DNA and RNA can be determined by using primers and probes OryzaDNA p1, OryzaDNA p2, OryzaDNA mb, OryzaRNA p1, OryzaRNA p2, OryzaRNA mb (table 1). During the application of herbicide (or other) compounds the conditions of the plants can be assessed by measurement of the chloroplast nucleic acid content of the cells using amplification methods like PCR and NASBA that are known to persons skilled in the art. At the same time, using primer sets suitable for weeds, the deterioration of the unwanted plants can be monitored. It is clear that these molecular tools are very suited in the research for new herbicides that specifically attack one group of plants and not others.

### Example 12

In this example the NASBA nucleic acid amplification reactions for DNA target sequences were performed in a 20µl reaction volume and contained: 40mM Tris-pH 8.5, 70mM KCl, 12mM MgCl2, 5mM dithiotreitol, 1mM dNTP's (each), 2mM rNTP's (each), 0.2µM primer (each), 0.05µM molecular beacon, 1.5 units restriction enzyme Msp I, 375mM sorbitol, 0.105 µg/µl bovine serum albumin, 6.4 units AMV RT, 32 units T7 RNA polymerase, 0.08 units RNAse H and input nucleic acid. The complete mixture, except the enzymes, sorbitol and bovine serum albumin was, prior to adding the enzyme mixture, incubated at 37°C for 25 minutes and subsequently heated to 95°C for two minutes in order to denature the DNA and to allow the primers to anneal. After cooling the mixture to 41°C the enzyme mixture was added. The amplification took place at 41°C for 90 min in a fluorimeter (CytoFluor 2000) and the fluorescent signal was measured every minute (using the filter set 530/25 nm and 485/30 nm).

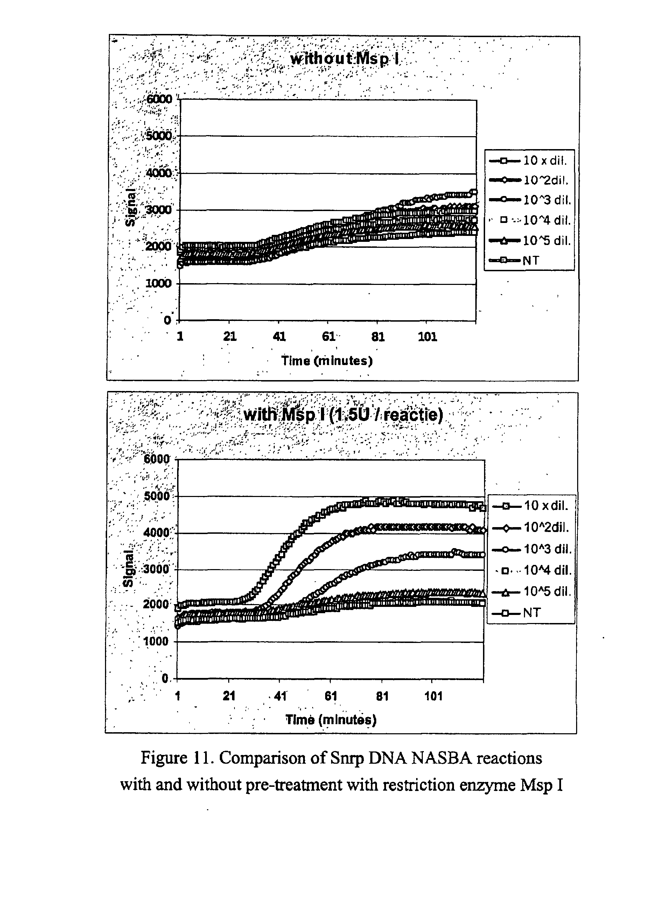

To achieve quantification, a dilution series of target sequence for a particular primer set was amplified and the time points at which the reactions became positive (the time to positivity, TTP) were plotted against the input amounts of nucleic acid. This way a calibration curve was created that could be used to read TTP values of reactions with unknown amounts of input and deduce the input amount. Fresh peripheral blood mononuclear cells (PBMC's) from a healthy blood donor were cultured *in vitro* for 5 days. After 5 days the cells were harvested and analyzed for amount of chromosomal DNA (primers SnrpD p1 and SnrpD2 p2 and probe SnrpD mb) with the described NASBA protocol in the chapter "Used ingredients and general methodology" and compared with the NASBA protocol as described in this example. As can be clearly seen in figure 11 the DNA NASBA reactions with pre-treatment of restriction enzyme perform much better than without. The rationale for this observation is the direct extension from the Msp I created 3' over the T7 promoter part of the p1 primer.

### Example 13

Using the primers and probes tRNA-L-D p1, tRNA-L-D p2, tRNA-L-D MB, petB RNA p1, petB RNA p2 and petB RNA MB (table 1) the chloroplast DNA and RNA of *Oryza sativum* (rice) can be quantified and the ratio to the chromosomal DNA and RNA can be determined by using primers and probes OryzaDNA p1, OryzaDNA p2, OryzaDNA mb, OryzaRNA p1, OryzaRNA p2, OryzaRNA mb (table 1). During the application of herbicide (or other) compounds the conditions of the plants can be assessed by measurement of the chloroplast nucleic acid content of the cells using amplification methods like PCR and NASBA that are known to persons skilled in the art. At the same time, using primer sets suitable for weeds, the deterioration of the unwanted plants can be monitored. It is clear that these molecular tools are very suited in the research for new herbicides that specifically attack one group of plants and not others.

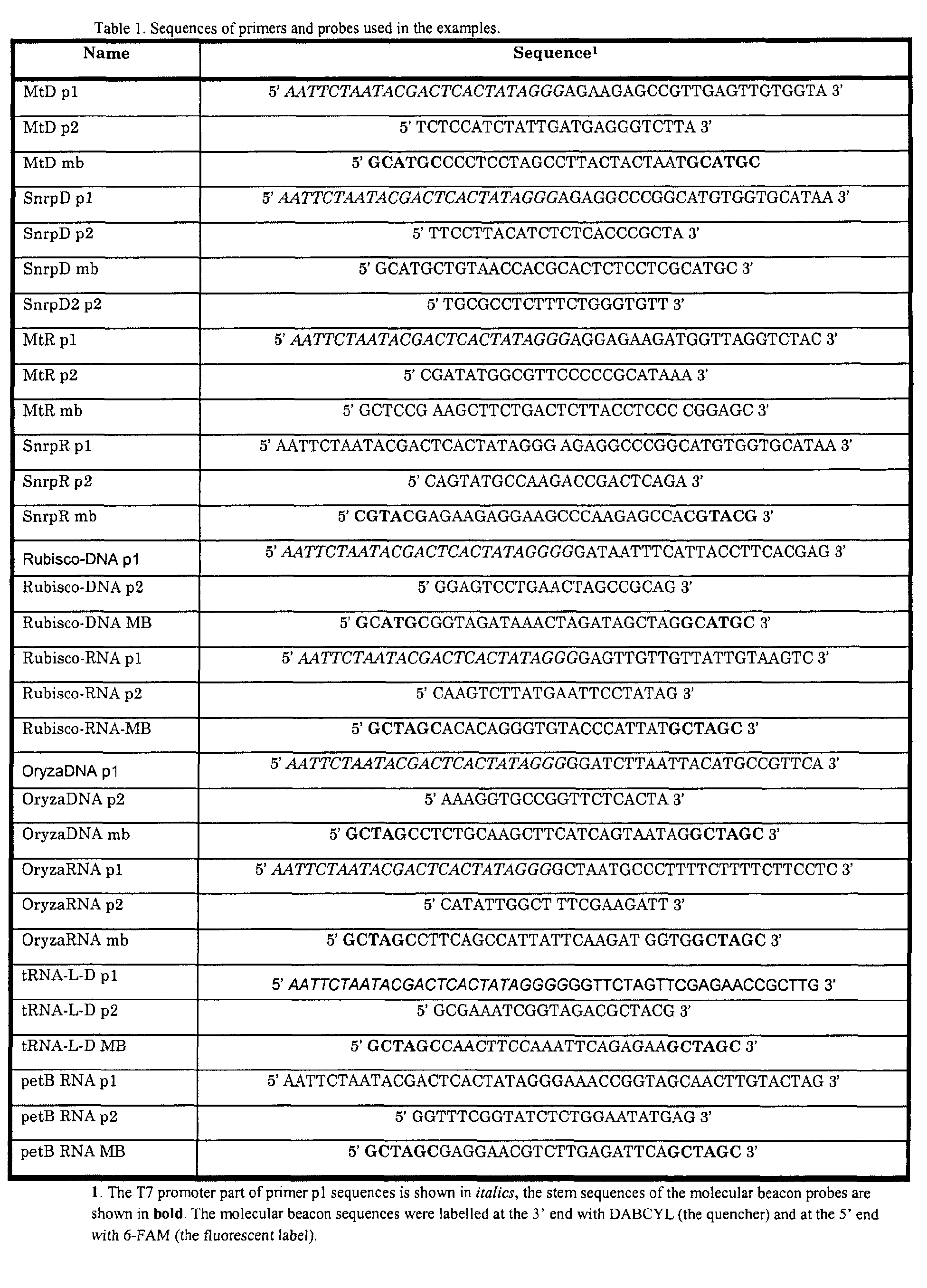

## Claims

1. A method for determining functioning of a cellular organism comprising determining the relative ratio of a first endosymbiont cellular organelle nucleic acid and/or gene product thereof in a sample obtained from said organism in relation to the amount of a second nucleic acid and/or gene product thereof.

2. A method according to claim 1 whereby said relative ratio of a first endosymbiont cellular organelle nucleic acid and/or gene product thereof is determined in relation to the amount of a nuclear nucleic acid and/or gene product thereof detectable in said sample.

3. A method according to claim 2 wherein said nuclear nucleic acid comprises DNA.

4. A method according to claim 3 wherein said nuclear nucleic acid comprises DNA encoding a component of a small nuclear ribonucleoprotein or fragment thereof.

5. A method according to claim-2 wherein said nuclear nucleic acid comprises RNA.

6. A method according to claim 5 wherein said nuclear nucleic acid comprises RNA encoding a component of a small nuclear ribonucleoprotein or fragment thereof.

7. A method according to anyone of claims 1 to 7 wherein said endosymbiont cellular organelle nucleic acid and/or or gene product thereof comprises RNA.

8. A method according to claim 1 whereby said relative ratio of a first endosymbiont cellular organelle nucleic acid and/or gene product thereof is determined in relation to the amount of a second endosymbiont cellular organelle nucleic acid and/or or gene product thereof detectable in said sample acid.

9. A method according to claim 8 wherein said first nucleic acid comprises DNA.

10. A method according to claim 8 wherein said first nucleic acid comprises RNA.

11. A method according to anyone of claims 1, 2 or 8 wherein said first nucleic acid comprises DNA and wherein said second nucleic acid comprises RNA.

12. A method according to claim 11 whereby said second nucleic acid is in essence derivable by transcription from said first nucleic acid.

13. A method for determining therapeutic activity and/or possible side-effects of a candidate compound in determining its usefulness for treatment of malfunctioning of a cellular organism, comprising determining the relative ratio of an endosymbiont cellular organelle nucleic acid and/or or gene product thereof in a sample obtained from an organism

14. A method according to claim 13 wherein said organism or an essentially related organism has been provided with said compound.

15. A method for determining toxic activity of a candidate compound in determining its usefulness for causing malfunctioning of a cellular organism, comprising determining the relative ratio of an endosymbiont cellular organelle nucleic acid and/or or gene product thereof in a sample obtained from an organism

16. A method according to claim 15 wherein said organism or an essentially related organism has been provided with said compound.

17. A method for determining selective activity of a candidate compound against a first organism comprising determining therapeutic activity and/or possible side-effects of a candidate compound with a method according to claim 13 or 14 and/or determining toxic activity with a method according to claim 15 or 16.

18. A method according to claim 17 further comprising providing an essentially unrelated second organism with said compound.

19. A method according to claim 18 wherein said first organism comprises a pathogen and said second organisms comprises a host for said pathogen.

20. A method according to claim 18 wherein said first organism comprises a weed plant and said second organism comprises a crop plant.

21. A diagnostic kit comprising at least one means for performing a method according to anyone of claims 1 to 18

22. A kit according to claim 21, comprising at least one primer or probe selective for the amplification and detection of a nucleic acid related to or derived from endosymbiont cellular organelles.

23. A kit according to claim 22 wherein said primer or probe is listed in Table 1.

24. Use of a compound obtainable or selectable by a method according to the invention in the preparation of a medicament, a herbicide, an insecticide, an anti-parasiticum, an cytostatic agent or cytotoxic agent.

25. A medicament, a herbicide, an insecticide, an anti-parasiticum, an cytostatic agent or cytotoxic agent obtainable or selectable by a method according to the invention.
